# EUROPÄISCHE PATENTSCHRIFT

(11) **EP 1 227 799 B1**
(45) Veröffentlichungstag und Bekanntmachung des Hinweises auf die Patenterteilung: **25.06.2003**
(21) Anmeldenummer: 00977481.1
(22) Anmeldetag: 03.11.2000
(51) Int. Cl.: A61K 9/20, A61K 9/70

(54) **EINE FILM-, FOLIEN- ODER OBLATENFÖRMIGE ZUBEREITUNG**
A FILM, SHEET OR WAFER-SHAPED PREPARATION
UN FILM, UNE FEUILLE OU UN CACHET D'ADMINISTRATION

(30) Priorität: 12.11.1999 DE 19954420
(43) Veröffentlichungstag der Anmeldung: 07.08.2002
(73) Patentinhaber: LTS Lohmann Therapie-Systeme AG, 56626 Andernach (DE)
(72) Erfinder: LUDWIG, Karin, 56589 Datzeroth (DE); KRUMME, Markus, 56567 Neuwied (DE)
(74) Vertreter: Flaccus, Rolf-Dieter, Dr.
(86) Internationale Anmeldenummer: EP0010862
(87) Internationale Veröffentlichungsnummer: WO01035931

(56) Entgegenhaltungen:
- EP-A- 0 398 229
- WO-A-95/09608
- WO-A-98/20859

## Beschreibung

Die Erfindung betrifft eine flächenförmige Zubereitung, bestehend aus einerlfilm-, folien- oder oblatenförmigen Darreichungsform, deren Aufbau zwei Schichten aufweist, welche zur Kennzeichnung der bei der Anwendung daraus freisetzbaren Substanzen mit einer Kodierung durch Schrift, graphische Zeichen oder Muster versehen sind. Die Zubereitung eignet sich insbesondere zur Verabreichung von geschmacksoder geruchswirksamen und/oder pflegenden bzw. heilenden Substanzen. Die Erfindung zeigt außerdem Verfahren auf, mittels derer die genannten Darreichungsformen mit Kodierungen versehen werden können.

Immer häufiger sind Hersteller pharmazeutischer Produkte verpflichtet, außer der Primärverpackung auch das Produkt als solches zu kennzeichnen. Dies betrifft insbesondere Medikamente in Darreichungsformen, mit denen eine gezielte Wirkstoffabgabe erzielt werden soll. Bei Medikamenten in Form von Dragees, Kapseln, bzw. folien- oder filmartigen Produkten erfolgt eine solche Kennzeichnung oder Kodierung nach dem Stand der Technik üblicherweise in einem zusätzlichen Arbeitsgang im Anschluß an die eigentliche Herstellung, und zwar durch Bedrucken mit oder Einstanzen von speziellen Kennzeichen oder Mustern.
Abgesehen von dem dadurch erforderlichen Aufwand mit entsprechend erhöhten Herstellungskosten ist die Originalität und Fälschungssicherheit insbesondere im Falle des Bedrukkens nicht besonders hoch, da solche Kennzeichnungen auch nachträglich relativ einfach anzubringen bzw. zu entfernen sind.
Die Bedruckung hat noch den weiteren Nachteil, daß sie ausschließlich der Kennzeichnung dient, jedoch keine weitere Funktion beispielsweise im Sinne einer Freisetzungsmodifikation oder einer Erhöhung der möglichen Beladung hat, und deshalb relativ teuer ist.

Bei Tabletten sind Verfahren zur Kennzeichnung bekannt, mit denen während der Herstellung reliefartige Zeichen oder Muster erzeugt werden. Gemäß den in der US-PS 5 516 530 bzw. der WO 95/09608 beschriebenen Verfahren wird das Tablettenmaterial als Dispersion in eine Form gegossen, in welcher die Zeichen oder Muster vorgebildet sind. Nach Erstarren der Masse, unter Anwendung von Druck, Gefrieren oder Gefriertrocknen, erhält man eine Tablette, welche die vorbestimmte reliefartige Prägung aufweist. Es ist leicht einzusehen, daß diese Methode zur Kennzeichnung von film-, folien- oder oblatenförmigen Darreichungsformen nicht geeignet ist.

Die DE-OS 196 46 836 A1 beschreibt einschichtige Darreichungsformen der letztgenannten Art, welche mit einer Kennzeichnung oder Kodierung versehen sind. Dabei handelt es sich um Dickenunterschiede, d. h. Vertiefungen und Erhebungen, in den flächigen Zubereitungen. Dadurch wird ein visueller Effekt hervorgerufen, der mit Wasserzeichen vergleichbar ist. Zur Erzeugung dieser Muster wird die Zubereitungsmasse auf eine entsprechend strukturierte Unterlage beschichtet, oder die Muster werden nachträglich unter Druckeinwirkung angebracht.
Die beschriebene Art der Kennzeichnung ist allerdings mit dem Nachteil behaftet, daß sie nicht besonders deutlich in Erscheinung tritt. Dies liegt vor allem daran, daß sie nur einfarbig ist und nur aufgrund geringfügiger Hell-Dunkel-Kontraste an den Konturen zu erkennen ist. Hinzu kommt, daß die Dickenunterschiede, welche dieses Wasserzeichenähnliche Muster erkennbar machen, sich nur im µm-Bereich bewegen. Bei den genannten dünnen Darreichungsformen ist es nicht möglich, die Dickenunterschiede zwecks Verbesserung der Erkennbarkeit weiter zu vergrößern.

Ein weiterer nicht zu unterschätzender Nachteil der in der DE-OS 196 46 836 A1 offenbarten Darreichungsformen besteht darin, daß durch das Anbringen der Muster in Gestalt von Vertiefungen insgesamt die mittlere Schichtdicke - und damit auch die maximal mögliche Beladung mit Wirkstoffen - reduziert wird. Bei Arzneiformen, die aufgrund ihrer Dimensionen ohnehin nicht hoch beladbar sind, z. B. filmförmige Darreichungsformen, fällt dieser Nachteil besonders ins Gewicht.

Aufgabe der vorliegenden Erfindung war es deshalb, eine Zubereitung mit den im Oberbegriff des Anspruchs 1 genannten Merkmalen anzugeben, welche mit einer Kennzeichnung oder Kodierung in Gestalt von Schrift, Zeichen oder Mustern versehen ist, jedoch nicht die Nachteile der aus dem Stand der Technik bekannten Darreichungsformen aufweist. Ferner bestand die Aufgabe darin, Verfahren anzugeben, mit denen die erfindungsgemäßen Darreichungsformen auf einfache und kostengünstige Weise hergestellt werden können.

Überraschenderweise gelingt die Lösung der Aufgabe dadurch, daß flächenförmige, insbesondere film-, folien- oder oblatenförmige Zubereitungen, einen zwei- oder mehrschichtigen Aufbau aufweisen, wobei zwei benachbarte Schichten unterschiedlich gefärbt sind und Flächenbereiche unterschiedlicher Dicke aufweisen, die zueinander komplementär (oder reziprok) sind.

Die Bezeichnung "unterschiedliche Dicke" bezieht sich auf Dickenunterschiede innerhalb jeweils einer der beiden Schichten. Deshalb können die erfindungsgemäßen zweischichtigen Zubereitungen auch Flächenbereiche aufweisen, in denen die beiden Schichten gleich dick oder annähernd gleich dick sind.

Durch die musterförmig verlaufenden Dickenunterschiede in den Schichten der film-, folien- oder oblatenförmigen Darreichungsform wird ein dem Auge sichtbares Kennzeichen im Flächenbereich der Zubereitung erzeugt, das in der Wirkung einem Wasserzeichen bei papierartigen Dispersionen ähnelt. Aufgrund der besonderen Anordnung zweier komplementär geformter und unterschiedlich gefärbter Schichten wird die visuelle Erkennbarkeit der durch die Dickenunterschiede verkörperten Zeichen oder Muster wesentlich verbessert. Ein weiterer Vorteil ergibt sich aus den vielfältigen Farbkombinationen, wobei durch eine geeignete Auswahl (z. B. Komplementärfarben) die Erkennbarkeit weiter gesteigert werden kann. Um einen optimalen visuellen Eindruck hervorzurufen, kann es von Vorteil sein, wenn die Schichten im wesentlichen opak sind. In einzelnen Fällen kann auch die Verwendung transparenter Schichten vorteilhaft sein. Bei den anzubringenden Kennzeichen kann es sich um Buchstaben, Zahlen, Symbole, Logos, Piktogramme oder bildliche Darstellungen handeln.

Die unterschiedliche Färbung der beiden Schichten kann durch Unterschiede hinsichtlich des Farbtons, der Farbsättigung oder der Farbhelligkeit, oder Kombinationen dieser Parameter, bewirkt werden. Auch die Farben Schwarz, Weiß sowie Grautöne werden als Farben im Sinne der Erfindung verstanden. Ferner werden auch Ausführungsformen umfaßt, bei denen eine der beiden Schichten klar-transparent ausgestaltet ist. Da der visuelle Effekt vor allem durch Lichtbrechung an den Phasengrenzen der beiden Schichten zustande kommt, können bereits geringfügige Unterschiede im Farbton, in der Farbsättigung oder der Farbhelligkeit bewirken, daß die Konturen der als Kodierung dienenden Kennzeichen oder Muster deutlich wahrnehmbar sind. Darüber hinaus heben sich auch die innerhalb bzw. außerhalb der Konturen befindlichen Flächenbereiche der Kennzeichen, Symbole oder Muster aufgrund des abweichenden Farbtons, oder der Farbsättigung oder der Farbhelligkeit, voneinander ab. Außerdem entstehen durch die Überlagerung zweier Flächenbereiche unterschiedlicher Dicke und unterschiedlicher Färbung jeweils zwei neue Farben oder Farbtöne, die sich daraus ergeben, daß im Überlagerungsbereich entweder die eine oder die andere Schicht dünner oder dicker ist. Auf diese Weise wird die Wahrnehmbarkeit der als Dickenunterschiede in den beiden Schichten ausgestalteten Kennzeichen oder Muster noch verstärkt.

Im Vergleich zu herkömmlichen Wasserzeichen-ähnlichen Kennzeichnungen, deren Wahrnehmbarkeit lediglich auf minimalen Hell-Dunkel-Kontrasten beruht, macht sich die vorliegende Erfindung die vorteilhaften Eigenschaften von Farbkontrasten zunutze. Zusätzlich treten dabei auch Hell-Dunkel-Kontraste in Erscheinung, die zur Wahrnehmbarkeit der Kennzeichnungen beitragen.
Da es sich bei der erfindungsgemäßen Kennzeichnung - wie bei herkömmlichen Wasserzeichen-ähnlichen Kennzeichnungen oder Prägungen - ebenfalls um eine Kodierung handelt, die fester und untrennbarer Bestandteil der Arzneizubereitung selbst ist, weist sie auch die damit verbundenen Vorteile (z. B. Fälschungssicherheit) auf, welche bei anderen Kodierungsverfahren, vor allem beim Bedrucken, nicht gegeben sind.

Vor allem für Arzneiformen, die aus dünnen Schichten bestehen (z. B. film- oder oblatenartige Darreichungsformen), stellt die erfindungsgemäße Art der Kennzeichnung oder Kodierung eine vorteilhafte Alternative gegenüber den bekannten Methoden dar. Die Erzeugung von kennzeichnenden Mustern oder Symbolen durch das Zusammenwirken von zwei komplementären, unterschiedlich gefärbten Schichten verbessert nicht nur die Erkennbarkeit, sondern eröffnet vielfältige neue Variationsmöglichkeiten für die Darstellung.

Da die Schichtdicke über die gesamte Fläche im wesentlichen konstant ist, kann eine optimale Wirkstoffbeladung erfolgen. Der zweischichtige Aufbau gestattet ferner eine Beladung mit unterschiedlichen Wirkstoffen oder sonstigen unterschiedlichen Substanzen der im Oberbegriff des Anspruchs 1 genannten Art. Weitere Möglichkeiten ergeben sich daraus, daß die beiden Schichten auf unterschiedliche Weise mit Zusatzstoffen versehen werden können, die das Freisetzungsverhalten beeinflussen.
Für bestimmte Anwendungszwecke kann es sinnvoll oder erforderlich sein, die erfindungsgemäße zweischichtige Darreichungsform mit weiteren Schichten auszustatten. Um die Erkennbarkeit der Kodierung nicht zu stören, werden dabei Schichten bevorzugt, die im wesentlichen transparent sind.

Eine Ausführungsform der Erfindung sieht vor, daß die Flächenbereiche unterschiedlicher Dicke, insbesondere in der zuerst hergestellten Schicht, herstellungsbedingt unter Vermeidung einer unterschiedlichen Dichte oder Verdichtung ausgebildet sind. Dies wird vorzugsweise dadurch erreicht, daß die Flächenbereiche unterschiedlicher Dicke der ersten Schicht ohne Einwirkung von Druck ausgebildet werden. Nachfolgend wird die zweite Schicht auf die erste Schicht in der Weise aufgebracht, daß sie die dort vorgebildeten reliefartigen Dickenunterschiede komplementär ergänzt.

Nach einer anderen Ausführungsform der Erfindung weisen die Bereiche unterschiedlicher Dicke, insbesondere diejenigen der zuerst hergestellten Schicht, aufgrund des Herstellungsverfahrens eine unterschiedliche Dichte oder Verdichtung auf. Dies kann dadurch erreicht werden, daß die Zeichen bzw. Muster der Kodierung in der bereits vorgefertigten ersten Schicht unter Einwirkung von Druck angebracht werden.

Das erfindungsgemäße Verfahren zur Herstellung der Zubereitung sieht vor, daß das Einbringen der als Kodierung dienenden Zeichen oder Muster in die erste Schicht durch irreversible Deformation im plastisch verformbaren Zustand erfolgt.
Um die Bereiche unterschiedlicher Dicke ohne Einwirkung von Druck zu erzeugen, wird die zur Herstellung der Schicht dienende Masse auf eine Unterlage beschichtet, welche eine der Kodierung (Kennzeichen oder Muster) entsprechende Folge von Vertiefungen und Erhebungen aufweist. Solche Vertiefungen bzw. Erhebungen können beispielsweise auf einer druckplattenähnlichen Unterlage durch Aufbringen einer Radierung oder Ätzung aufgebracht sein.

Um die Bereiche unterschiedlicher Dicke der ersten Schicht unter Einwirkung von Druck zu erzeugen, können beispielsweise entsprechend strukturierte Prägewalzen oder Stempel verwendet werden.

Nach erfolgter Herstellung der ersten Schicht mit reliefartigen Bereichen unterschiedlicher Dicke, sei es mit oder ohne Einwirkung von Druck, wird die zweite Schicht durch Beschichten aus Lösung oder Schmelze aufgetragen, und zwar auf diejenige Seite der ersten Schicht, welche die reliefartigen Vertiefungen und Erhöhungen aufweist. Die zur Herstellung der zweiten Schicht verwendete Beschichtungsmasse muß so ausgewählt werden, daß sie einerseits eine gute Verankerung der zweiten Schicht auf der ersten, strukturierten Schicht ermöglicht, andererseits aber nicht die erste Schicht auflöst oder schmilzt.

Unter "Beschichten" wird jegliche Art des Aufbringens einer verfestigbaren flüssigen Masse auf einen Träger durch Rakel-, Walzenauftrags-, Sprüh-, Extrusions- oder Sprühverfahren verstanden.

Eine weitere erfindungsgemäße Methode, um die erste Schicht mit musterartigen Dickenunterschieden zu versehen, besteht darin, daß die flächenförmige Zubereitung während des Trocknens mit lokal unterschiedlichen Temperaturen beaufschlagt wird. Dies kann durch selektive Zufuhr von Heißluft erfolgen, wobei die zu trocknende Schicht sich auf einer Unterlage befindet, welche ein gitterartiges Muster aufweist oder aus unterschiedlich wärmeleitenden Materialien besteht, die so angeordnet sind, daß sie Muster oder Kennzeichen ausbilden können. Durch die Einwirkung lokaler Temperaturunterschiede werden Dickenunterschiede in der Schicht erzeugt, dergestalt, daß das Material in den wärmeren Zonen dicker und in den kühleren Zonen dünner ist. Die Temperaturunterschiede, die dabei zwischen den wärmeren und kälteren Zonen lokal auftreten können, liegen in einer Größenordnung von 10-100 °C, bevorzugt zwischen 30-80 °C.

Die erfindungsgemäßen Verfahren können kontinuierlich oder diskontinuierlich ausgestaltet sein. Eine diskontinuierliche Herstellung kann beispielsweise dadurch erfolgen, daß die plastisch verformbare Masse auf einem dehäsiv ausgerüsteten Förderband transportiert wird und dabei mittels einer strukturierten Prägewalze oder durch Beaufschlagung mit lokalen Temperaturunterschieden mit einem Kennzeichen oder Muster zur Kodierung versehen wird. Anschließend wird durch ein ebenfalls kontinuierliches Auftragsverfahren (Rakel, Walzen) die zweite Schicht aufgetragen.
Bei einem diskontinuierlichen oder intermittierenden Herstellungsverfahren kann die Beschichtungsmasse der ersten Schicht auf eine entsprechend strukturierte Unterlage mit einer vorgegebenen Fläche aufgetragen werden. Alternativ können die als Kodierung dienenden Kennzeichen, Muster oder Symbole auch mit einem Stempel in die plastisch verformbare erste Schicht eingebracht werden. Nach deren Verfestigung wird auf der mit reliefartigen Erhöhungen und Vertiefungen versehenen Schichtseite der ersten Schicht die Schichtmasse für die zweite Schicht mittels eines der genannten Beschichtungsverfahren aufgetragen.

Im folgenden wird die Herstellung kodierter, d. h. mit Dikkenunterschieden in Form von Kennzeichen, Mustern oder Symbolen versehener, flächenhafter Darreichungsformen anhand von Ausführungsbeispielen näher erläutert. Dabei werden mit I, II und III drei unterschiedliche Herstellungsverfahren bezeichnet. Diese sehen eine Abfolge von Arbeitsschritten A - E wie folgt vor:

### A. Herstellung der Schichtmasse

Vereinigen von Einzelkomponenten durch Mischen, Lösen, Dispergieren, Suspendieren oder Emulgieren. Als Löse-, Dispergierungs- oder Suspensionsmittel dient vorzugsweise ein Ethanol/Wassergemisch, jedoch können auch andere leicht entfernbare Lösemittel oder Lösemittelgemische eingesetzt werden.
Eine beispielhafte Formulierung einer Schichtmasse, die sich besonders für die Bildung von Flächenbereichen unterschiedlicher Dicke unter Einwirkung von Druck ("Prägeverfahren") eignet, ist:
32,8 Gew.-% Polyvinylpyrrolidon
11,5 Gew.-% Hydroxypropylcellulose
6,6 Gew.-% Titandioxyd
32,8 Gew.-% Siliciumdioxyd
16,4 Gew.-% Polyethylenglykol 400
und Ethanol als Lösungsmittel

Eine beispielhafte Formulierung einer Schichtmasse, die sich besonders für die Bildung von Flächenbereichen unterschiedlicher Dicke ohne Einwirkung von Druck (" thermisches Verfahren") eignet, ist:
28,6 Gew.-% Polyvinylalkohol
7,9 Gew.-% Titandioxyd
37,2 Gew.-% Siliciumdioxyd
11,5 Gew.-% Polyethylenglykol 400
4,6 Gew.-% Polyethylenglykol 4000
10,2 Gew.-% Sorbitol

### B. Beschichtung

Auftragsvorrichtung: Walzen, Streichkasten, Düsen. Kontinuierliche Beschichtung auf:
I. strukturierte Unterlage (z. B. Teflon, Edelstahl)
II. dehäsiv ausgerüstetes Material (z. B. Papier, Folie) mit planer Oberfläche
III. wie unter II.

### C. Trocknung

zu I.: mittels Heißluft

zu II.: mittels Heißluft, wobei "wärmere" und "kältere" Zonen vorhanden sein müssen.
Hierdurch entsteht ein folienartiges Band, das unterschiedliche Dicken aufweist. An den wärmeren Zonen ist das Material dicker, an den kälteren ist es dünner.
Diese Zonen werden erzielt durch
a) eine durchbrochene Oberfläche, z. B. Gitter-Lochstruktur der bandförmigen Unterlage
b) Verwendung von unterschiedlich wärmeleitenden Materialien bei der Herstellung des Transportbandes
c) dehäsiv ausgerüstetes Material, in welchem Segmente mit sich abgrenzenden wärmeleitenden Eigenschaften implantiert sind.

zu III.: Mittels Heißluft wie bei I. und zusätzlichem Einsatz einer strukturierten Prägewalze.

### D. Auftragen der zweiten Schicht

Die zweite Schicht wird durch Beschichten mit einer geeigneten Masse, die auch während des Beschichtungsprozesses die erste Schicht nicht auf- oder anlöst oder -schmilzt, auf der ersten, strukturierten Schicht verankert.

### E. Konfektionierung

1. Längsschneiden in Schmalrollen
2. Vereinzeln durch Stanzen oder Querschneiden (bei Verfahren II wird das Papier bzw. die Folie vorher entfernt.
3. Verpacken.

Weitere Einzelheiten, Merkmale und Vorteile der Erfindung ergeben sich aus der nachstehenden Erläuterung der in den Zeichnungen schematisch dargestellten Ausführungsbeispiele. Es zeigen:
Fig. 1 eine schematische Darstellung der fertigen, mit Kodierung versehenen zweischichtigen Arzneiform (im Schnitt),
Fig. 2 eine schematische Darstellung (im Schnitt) mit Herstellung der Kodierung in der ersten Schicht auf einer mit Dickenunterschieden versehenen Unterlage,
Fig. 3 die Herstellung einer Kodierung in der ersten Schicht durch Beaufschlagung der Zubereitung mit lokalen Temperaturunterschieden (im Schnitt).

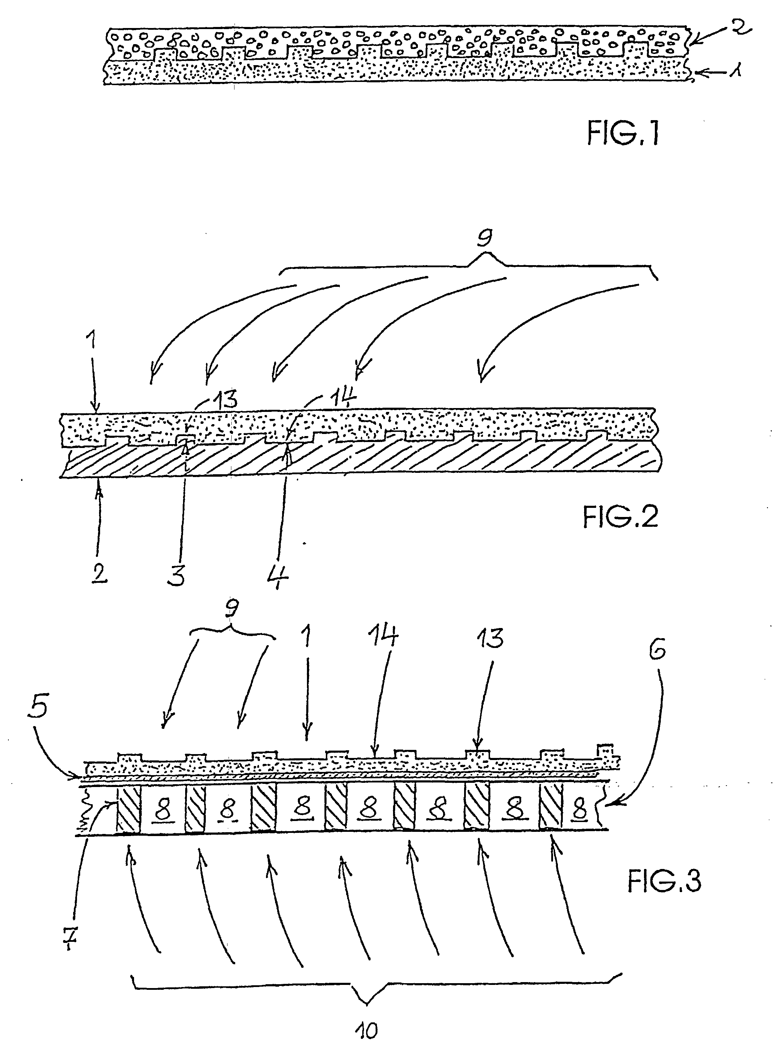

Fig. 1 zeigt die zweischichtige Zubereitung mit der strukturierten ersten Schicht (1) und der reziproken (=komplementären) zweiten Schicht (2), wobei die Gesamtdicke des Systems an jeder Stelle konstant ist.

Fig. 2 zeigt die erste Schicht (1) der Zubereitung auf einer strukturierten Unterlage (2), welche eine Folge von Erhebungen (3) und Vertiefungen (4) aufweist. Entsprechend entstehen reziprok zu den Erhebungen (3) Vertiefungen (13) und zu den Vertiefungen (4) Erhebungen (14) in der ersten Schicht (1) der Zubereitung. Die Wärmezuführung erfolgt in einer Wärmeströmung (9), bei welcher es sich sowohl um Strahlungswärme als auch um Konvektion handeln kann.

Fig. 3 zeigt ein Herstellungsverfahren, bei welchem die Dickenunterschiede (13, 14) in der Schicht (1) der Zubereitung beim Trocknen mit lokalen Temperaturunterschieden hergestellt werden. Dabei liegt die erste Schicht (1) der Zubereitung auf einem folienförmigen Träger (5), beispielsweise Silikonpapier, Alufolie, Kunststoffolie oder dergleichen. Mit (6) ist ein Transportband bezeichnet, welches eine aus Stegen (7) und Durchbrüchen (8) bestehende Gitteroder Lochstruktur aufweist. Durch Wärmezufuhr an die Stege entsprechend den Pfeilen (10) erzeugen diese im Kontakt mit der Trägerfolie (5) wärmere Bereiche und im Bereich der Durchbrüche (8) kältere Bereiche. Im Bereich der wärmeren Zonen fällt das Material bei der Trocknung dicker aus, während es in den kälteren Bereichen dünner ist.

Dabei kann der Trocknungsvorgang von oben her beispielsweise von einem Strom (9) angewärmter Luft unterstützt werden. Die Temperaturunterschiede des Transportbandes (6) können beispielsweise durch induktive Aufheizung der Stege (7) der Gitterstruktur oder durch Strablungs- oder Kontaktheizung erzeugt werden, während die Durchbrüche (8) beispielsweise mit Luft konvektiv gekühlt werden.

Die Erfindung ist unkompliziert auszuführen und erfüllt in optimaler Weise die eingangs gestellte Aufgabe.

## Patentansprüche

1. Film-, folien- oder oblatenförmige Zubereitung zur Verabreichung von geschmacks- oder geruchswirksamen und/oder pflegenden und/oder heilenden Substanzen, mit einer Kodierung dieser Substanzen durch Schrift- und/oder graphische Zeichen oder Muster, wobei die Kodierung durch Flächenbereiche unterschiedlicher Dicke ausgebildet ist,
**dadurch gekennzeichnet, daß** die Zubereitung zwei unterschiedlich gefärbte benachbarte Schichten umfaßt, welche zueinander komplementäre Flächenbereiche unterschiedlicher Dicke aufweisen.

2. Zubereitung nach Anspruch 1, **dadurch gekennzeichnet, daß** die Schichten im wesentlichen opak gefärbt sind.

3. Zubereitung nach Anspruch 1 oder 2, **dadurch gekennzeichnet, daß** sie eine oder mehrere zusätzliche Schichten aufweist, die im wesentlichen transparent sind.

4. Zubereitung nach einem der Ansprüche 1 bis 3, **dadurch gekennzeichnet, daß** die Flächenbereiche unterschiedlicher Dicke herstellungsbedingt unter Vermeidung einer unterschiedlichen Dichte oder Verdichtung ausgebildet sind.

5. Zubereitung nach einem der Ansprüche 1 bis 3, **dadurch gekennzeichnet, daß** die Flächenbereiche unterschiedlicher Dicke eine unterschiedliche Dichte besitzen.

6. Verfahren zur Herstellung einer Zubereitung nach einem der Ansprüche 1 bis 4, **dadurch gekennzeichnet, daß** die Flächenbereiche unterschiedlicher Dicke der ersten Schicht ohne Einwirkung von Druck ausgebildet werden.

7. Verfahren nach Anspruch 6, **dadurch gekennzeichnet, daß** die Dickenunterschiede der ersten Schicht durch Beschichten ainer Unterlage hergestellt werden, die eine der Kodierung entsprechende Folge von Erhebungen oder Vertiefungen aufweist.

8. Verfahren nach Anspruch 6, **dadurch gekennzeichnet, daß** die Dickenunterschiede der ersten Schicht durch vorbestimmte lokale Temperaturunterschiede beim Trocknen der Zubereitung erzeugt werden.

9. Verfahren zur Herstellung einer Zubereitung nach Anspruch 1, 2, 3 oder 5, **dadurch gekennzeichnet, daß** die Flächenbereiche unterschiedlicher Dicke der ersten Schicht unter Einwirkung von Druck ausgebildet werden.

10. Verfahren zur Herstellung einer Zubereitung nach Anspruch 1, 2, 3 oder 5, **dadurch gekennzeichnet, daß** die Kodierung der Flächenbereiche mit Zeichen oder Mustern in einem plastisch verformbaren Zustand der Zubereitung erfolgt.

11. Verfahren nach einem der Ansprüche 6 bis 10, **dadurch gekennzeichnet, daß** die Dickenunterschiede auf diskontinuierliche Weise in die Schicht eingebracht werden.

12. Verfahren nach einem der Ansprüche 6 bis 10, **dadurch gekennzeichnet, daß** die Dickenunterschiede auf kontinuierliche Weise in die Schicht eingebracht werden.

13. Verfahren zur Herstellung einer Zubereitung nach einem oder mehreren der Ansprüche 1 bis 5, **dadurch gekennzeichnet, daß** die komplementäre zweite Schicht durch Beschichten der ersten, nach einem der Ansprüche 6 bis 12 mit Dickenunterschieden versehenen Schicht mit einer geeigneten Masse mittels Rakel-, Walzenauftrags-, Sprüh- oder Extrusionsverfahren und nachfolgender Verfestigung erzeugt wird.

## Claims

1. Sheet-like, film-like or wafer-like preparation for administering substances affecting flavour or odour and/or substances for care and/or curative substances, comprising a coding of these substances in the form of characters and/or graphic symbols or patterns, with the coding being formed by surface areas of differing thickness, **characterized in that** the preparation comprises two differently coloured, adjacent layers which have surface areas of differing thickness which are complementary to each other.

2. Preparation according to Claim 1, **characterized in that** the layers are of a substantially opaque colour.

3. Preparation according to Claim 1 or 2, **characterized in that** it comprises one or two additional layers which are substantially transparent.

4. Preparation according to any one of Claims 1 to 3, **characterized in that** the surface areas of differing thickness are formed under the production conditions while avoiding differences in density or compression.

5. Preparation according to any one of Claims 1 to 3, **characterized in that** the surface areas of differing thickness have a different density.

6. Method for producing a preparation according to any one of Claims 1 to 4, **characterized in that** the surface areas of differing thickness of the first layer are formed without the action of pressure.

7. Method according to Claim 6, **characterized in that** the thickness differences of the first layer are produced by coating of a support which has a sequence of elevations or depressions corresponding to the coding.

8. Method according to Claim 6, **characterized in that** the thickness differences of the first layer are produced by predetermined local temperature differences during drying of the preparation.

9. Method for producing a preparation according to Claim 1, 2, 3 or 5, **characterized in that** the surface areas of differing thickness of the first layer are formed under the action of pressure.

10. Method for producing a preparation according to Claim 1, 2, 3 or 5, **characterized in that** the coding of the surface areas with symbols, characters or patterns takes place with the preparation in a plastically deformable state.

11. Method according to any one of the Claims 6 to 10, **characterized in that** the thickness differences are introduced into the layer in a discontinuous manner.

12. Method according to any one of Claims 6 to 10, **characterized in that** the thickness differences are introduced into the layer in a continuous manner.

13. Method for producing a preparation according to one or more of Claims 1 to 5, **characterized in that** the complementary second layer is produced by coating the first layer, which has been provided with thickness gradients according to one of Claims 6 to 12, with a suitable mass by means of a knife application process, roller application process, spraying process or extrusion process, and subsequent hardening.

## Revendications

1. Préparation sous forme de film, de feuille ou de cachet pour l'administration de substances agissant sur le goût ou l'odeur et/ou traitantes et/ou curatives, comportant un codage de ces substances par des caractères écrits et/ou éléments graphiques ou motifs, dans laquelle le codage est configuré par des zones de surfaces d'épaisseur différente,
**caractérisée en ce que** la préparation comprend deux couches adjacentes colorées différemment, qui présentent des zones de surfaces, complémentaires les unes des autres, d'épaisseur différente.

2. Préparation selon la revendication 1, **caractérisée en ce que** les couches sont colorées essentiellement de manière opaque.

3. Préparation selon la revendication 1 ou 2, **caractérisée en ce qu**'elle présente une ou plusieurs couches supplémentaires qui sont essentiellement transparentes.

4. Préparation selon l'une quelconque des revendications 1 à 3, **caractérisée en ce que** les zones de surfaces d'épaisseur différente sont conçues, en raison de la fabrication, en évitant une densité ou une compression différente.

5. Préparation selon l'une quelconque des revendications 1 à 3, **caractérisée en ce que** les zones de surfaces d'épaisseur différente possèdent une densité différente.

6. Procédé de fabrication d'une préparation selon l'une quelconque des revendications 1 à 4, **caractérisé en ce que** les zones de surfaces d'épaisseur différente de la première couche sont conçues sans l'action d'une pression.

7. Procédé selon la revendication 6, **caractérisé en ce que** les différences d'épaisseur de la première couche sont réalisées par enduction d'un support qui présente une suite, correspondant au codage, de reliefs ou de creux.

8. Procédé selon la revendication 6, **caractérisé en ce que** les différences d'épaisseur de la première couche sont produites par différences locales prédéterminées de température lors du séchage de la préparation.

9. Procédé de fabrication d'une préparation selon la revendication 1, 2, 3 ou 5, **caractérisé en ce que** les zones de surfaces d'épaisseur différente de la première couche sont conçues sous l'action d'une pression.

10. Procédé de fabrication d'une préparation selon la revendication 1, 2, 3 ou 5, **caractérisé en ce que** le codage des zones de surfaces avec des caractères ou des motifs est effectué dans un état pouvant subir une déformation plastique de la préparation.

11. Procédé selon l'une quelconque des revendications 6 à 10, **caractérisé en ce que** les différences d'épaisseur sont introduites de manière discontinue dans la couche.

12. Procédé selon l'une quelconque des revendications 6 à 10, **caractérisé en ce que** les différences d'épaisseur sont introduites de manière continue dans la couche.

13. Procédé de fabrication d'une préparation selon l'une quelconque ou plusieurs des revendications 1 à 5, **caractérisé en ce que** la deuxième couche complémentaire est produite par enduction de la première couche, munie de différences d'épaisseur selon l'une quelconque des revendications 6 à 12, par une masse appropriée au moyen d'un procédé de raclage, d'application au rouleau, de pulvérisation ou d'extrusion, et par durcissement subséquent.
